# EUROPEAN PATENT APPLICATION

(11) **EP 4 797 222 A1**
(43) Date of publication of application: **26.08.2026**
(21) Application number: 26158107.8
(22) Date of filing: 12.02.2026
(51) Int. Cl.: G06V 10/82, G06V 20/69, G16H 30/40, G16H 50/20

(54) **METHOD AND APPARATUS FOR ANALYZING PATHOLOGICAL SLIDE IMAGE**

(30) Priority: 19.02.2025 KR 20250021609; 24.12.2025 KR 20250210115
(71) Applicant: Lunit Inc., Seoul 06241 (KR)
(72) Inventor: OH, Jin Woo, 06241 Seoul (KR); LEE, Seungeun, 06241 Seoul (KR); WANG, Soohyun, 06241 Seoul (KR); HWANG, Woochan, 06241 Seoul (KR)
(74) Representative: AWA Sweden AB

(57) **Abstract**

A computing device according to an aspect includes at least one memory in which at least one command is stored, and at least one processor operating according to the at least one command, wherein the at least one processor is configured to generate information about cells and components of the cells expressed in a pathological slide image by analyzing the pathological slide image by using a machine learning model, extract at least one feature for the cells and the components based on the generated information, and control a display device to output information about the at least one feature.

## Description

### BACKGROUND

### 1. Field

The present disclosure relates to a method and apparatus for analyzing a pathological slide image. More specifically, the present disclosure relates to a method and apparatus for extracting features of cells and components thereof expressed in a pathological slide image by using a machine learning model and providing interpretable medical information based on the features.

### 2. Description of the Related Art

The field of digital pathology is a field in which histological information of a corresponding patient is acquired or a prognosis is predicted by using a whole slide image (WSI) generated by scanning a pathological slide image.

Recent machine learning models have achieved high accuracy in tasks such as cancer detection, cell subtype classification, or quantification of staining intensity based on analysis of pathological slide images. In addition, machine learning models may directly learn complex patterns from histopathological slides (for example, hematoxylin and eosin (H&E)-stained slides or immunohistochemistry (IHC)-stained slides).

### SUMMARY

The present disclosure is directed to providing an apparatus, a method, and a computer program which are capable of alleviating a black-box problem of a machine learning model, enhancing interpretability of a pathological slide image, and quantitatively analyzing subtle subcellular staining patterns.

The present disclosure is also directed to deriving meaningful medical information without a large-scale specialized labeling dataset and providing an analysis tool usable for improving existing machine learning models or performing quality control (QC).

The technical problems to be solved are not limited to the above-described technical problems, and other technical problems may exist.

A computing device according to an aspect includes at least one memory in which at least one command is stored, and at least one processor operating according to the at least one command, wherein the at least one processor is configured to generate information about cells and components of the cells expressed in a pathological slide image by analyzing the pathological slide image by using a machine learning model, extract at least one feature for the cells and the components based on the generated information, and control a display device to output information about the at least one feature.

A method of analyzing a pathological slide image according to another aspect includes generating information about cells and components of the cells expressed in a pathological slide image by analyzing the pathological slide image by using a machine learning model, extracting at least one feature for the cells and the components based on the generated information, and outputting information about at least one feature.

A computer-readable recording medium according to another aspect includes a recording medium having recorded thereon a program for causing the method to be executed on a computer.

### BRIEF DESCRIPTION OF THE DRAWINGS

The above and other aspects, features, and advantages of certain embodiments of the disclosure will be more apparent from the following description taken in conjunction with the accompanying drawings in which:
FIG. 1 is a diagram for describing an example in which a computing device according to an embodiment analyzes a pathological slide image;
FIG. 2A is a block diagram illustrating an example of a user terminal according to an embodiment;
FIG. 2B is a block diagram illustrating an example of a server according to an embodiment;
FIG. 3 is a flowchart for describing an example of a method of analyzing a pathological slide image according to an embodiment;
FIG. 4 is a diagram for describing an example in which a processor according to an embodiment analyzes a pathological slide image;
FIG. 5 is a diagram for describing an example in which the processor according to an embodiment generates information about cells and components of the cells;
FIG. 6 is a diagram for describing an example in which the processor according to an embodiment extracts features for cells and components;
FIG. 7 is a flowchart for describing an example in which the processor according to an embodiment outputs medical information about a subject;
FIG. 8 is a diagram for describing an example in which the processor according to an embodiment outputs medical information;
FIG. 9A is a diagram for describing another example in which the processor according to an embodiment outputs medical information;
FIG. 9B is a diagram for describing another example in which the processor according to an embodiment outputs medical information;
FIG. 10 is an image for describing another example in which the processor according to an embodiment outputs medical information;
FIG. 11 is a diagram for describing another example in which the processor according to an embodiment outputs medical information;
FIG. 12 is a diagram for describing another example in which the processor according to an embodiment outputs medical information; and
FIG. 13 is a diagram for describing an example of a system for outputting medical information according to an embodiment.

### DETAILED DESCRIPTION

Although terms used herein are selected from among general terms that are currently and widely used in consideration of functions in embodiments, these may be changed according to intentions or customs of those skilled in the art or the advent of new technology. In addition, in specific cases, terms intentionally selected by the applicant may be used, and in this case, the meaning of the terms will be disclosed in corresponding description of the present disclosure. Therefore, the terms used herein should be defined based on the overall content of the present disclosure instead of a simple name of each of the terms.

Throughout the specification, unless explicitly described to the contrary, the terms "include" and "including" will be understood to imply the inclusion of stated elements rather than the exclusion of any other elements. In addition, the term "unit," "module," or the like implies a unit of processing at least one function or operation and may be implemented in hardware or software or in combination of the hardware and the software.

In addition, terms "ordinal numbers" such as "first" and "second" may be used to describe various components, but the components should not be limited by the terms. The above terms are used only for distinguishing one constituent element from other constituent elements.

Hereinafter, the term "medical information" may refer to any medically meaningful information or clinical information of a subject that may be extracted from medical images. Medical images may include pathological slide images as well as radiological images (X-ray, computed tomography (CT), magnetic resonance imaging (MRI), or positron emission tomography (PET) images). For example, the medical information may include at least one of an immune phenotype, a genotype, an expressome, a biomarker, tumor purity, RNA-related information, a tumor microenvironment, a treatment regimen for cancer expressed in a pathological slide image, survival information, a treatment response, a treatment result, genetic characteristics, and medical records.

In addition, the medical information may further include anatomical structure information extracted from medical images, types of lesions, locations and sizes of lesions, morphological features of lesions (for example, boundaries, textures, and densities), functional indicators (for example, blood flow and metabolic activity), abnormal findings of organs, prognosis-related indicators obtained from medical images, information about findings acquired by analyzing medical images through an artificial intelligence (AI) model, abnormality scores of the findings, reliability of the findings, and image biomarkers (radiomic features).

In addition, the medical information may include findings such as the presence or absence of nodules in medical images, findings of pneumonia, presence of pneumothorax, locations and shapes of fractures, locations, sizes, shapes, and boundary characteristics of masses, distributions of microcalcifications, asymmetric lesions, density of breast tissue, and structural distortions. Such findings may be produced together with an abnormality score or risk score for a corresponding image, but one or more embodiments are not limited thereto.

In addition, the medical information may include areas, locations, and sizes of specific tissues (for example, cancer tissue and cancer stromal tissue) and/or specific cells (for example, tumor cells, lymphocyte cells, macrophage cells, endothelial cells, and fibroblast cells) in medical images, cancer diagnosis information, information related to a subject's likelihood of developing cancer, and/or medical conclusions associated with cancer treatment, but one or more embodiments are not limited thereto.

In addition, the medical information may include not only quantitative values obtainable from medical images, but also visualized information of the values, predictive information based on the values, image information, and statistical information. For example, the medical information may be provided to a user terminal or output through a display device.

Hereinafter, embodiments will be described in detail with reference to the accompanying drawings. However, the embodiments may be implemented in various forms and are not limited to the examples described herein.

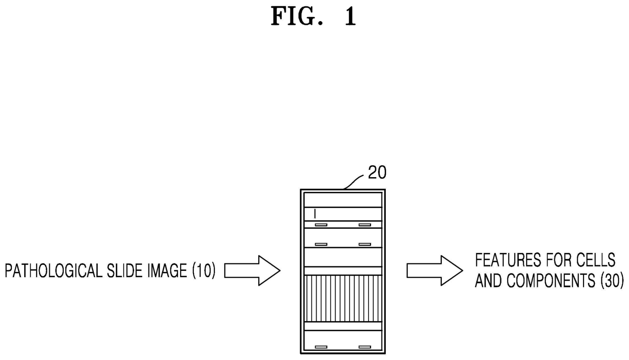

FIG. 1 is a diagram for describing an example in which a computing device 20 according to an embodiment analyzes a pathological slide image 10.

Referring to FIG. 1, the computing device 20 may analyze the pathological slide image 10 to output features 30 for cells and components.

For example, the computing device 20 may receive the pathological slide image 10 of a subject as an input. The computing device 20 may analyze the pathological slide image 10 by using a machine learning model and generate the features 30 for cells and components based on an analysis result. Here, the components may include a cell membrane, cytoplasm, and a nucleus.

A machine learning model refers to a statistical learning algorithm implemented based on a structure of a biological neural network or a structure configured to execute the algorithm. For example, the machine learning model may represent a model that has a problem-solving ability by nodes, which are artificial neurons forming a network through a synaptic coupling as in a biological neural network, repeatedly adjusting synaptic weights and learning such that an error between a correct output corresponding to a specific input and an inferred output is reduced. For example, the machine learning model may include any probabilistic model, neural network model, or the like used in Al learning methods such as deep learning.

For example, the machine learning model may be implemented as a multilayer perceptron (MLP) which includes multiple layers of nodes and connections therebetween. A machine learning model according to the present embodiment may be implemented by using one of various artificial neural network model structures including an MLP. For example, the machine learning model may include an input layer that receives input signals or data from the outside, an output layer that outputs output signals or data corresponding to the input data, and at least one hidden layer that is located between the input layer and the output layer, receives signals from the input layer, and extracts characteristics to transmits the extracted characteristics to the output layer. The output layer receives signals or data from the hidden layer and outputs the received signals or data to the outside.

Accordingly, the machine learning model may be trained to generate information about one or more objects (for example, cells, components of the cells, tissues, or structures) included in the pathological slide image 10. In particular, the machine learning model may be trained to extract features for cells and components.

Machine learning models according to a related art tend to operate as black boxes with limitations for users to directly interpret a pathological slide image. In addition, the machine learning models according to the related art have limitations in that the machine learning models are unable to perform nuanced pattern characterization on the pathological slide image 10 and are limited to analysis at a whole-image level or a cellular level.

For example, in lung cancer, an anaplastic lymphoma kinase (ALK) protein immunohistochemistry (IHC) may exhibit a strong granular cytoplasmic staining pattern in true-positive tumor cells. Patterns (for example, weakly diffused cytoplasmic staining or localized perinuclear dot-like patterns) different from those described above may be technical errors or represent specific molecular variants.

As described above, subtle patterns (for example, a granular texture, membrane accentuation, or speckled or vesicular localization) observed in the pathological slide image 10 have biological significance. Nevertheless, the machine learning models according to the related art are unable to explicitly identify or quantify the subcellular details described above.

In addition, developing end-to-end Al solutions for each of new pathological tasks may be impractical. Specifically, in order to train a robust deep learning model, a large-scale annotated dataset is required. However, the large-scale annotated dataset is difficult to obtain in the field of pathology. That is, unlike datasets of general images, pathology data requires expert labeling at a pixel level or cellular level, and such a process is labor-intensive and costly.

The computing device 20 according to an embodiment analyzes the pathological slide image 10 by using the machine learning model and extracts the features 30 for cells and components. That is, the computing device 20 may construct an interpretable feature library specialized in immunohistochemistry (IHC). For example, the feature library may include staining intensity, a texture, and spatial patterns at a subcellular level. The computing device 20 may use a library to characterize tissue in a standardized manner.

Such a feature panel (that is, the feature library) may be applicable to various types of IHC experiments or studies and may be used as a hypothesis generation or verification tool. For example, a user may apply the feature panel to clinical trial samples to analyze whether a specific pattern is correlated with drug responsiveness. For example, a user may apply the feature panel to a plurality of tissue microarrays to identify patterns that contribute to distinguishing subsets of subjects.

Accordingly, a black box problem observed in machine learning models according to a related art may be alleviated, and the interpretability of pathological slide images may be improved. In addition, subtle subcellular staining patterns in the pathological slide image 10 may be quantitatively identified.

In addition, the computing device 20 may derive meaningful medical information from the pathological slide image 10 without a large-scale specialized labeling dataset. In addition, the computing device 20 may provide an analysis tool that may be used for improving existing machine learning models or performing quality control (QC).

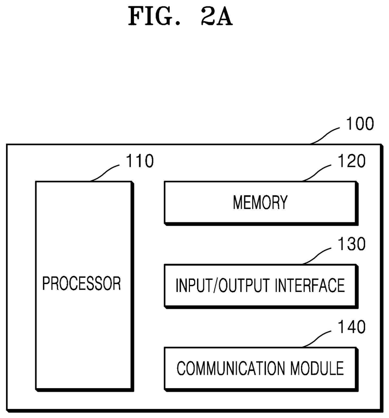

Hereinafter, examples in which the computing device 20 analyzes the pathological slide image 10 and extracts the features 30 will be described with reference to FIGS. 2A to 12.

For example, the computing device 20 may be a user terminal or a server. In other words, operations performed by the computing device 20 may be performed by the user terminal or the server. For example, some of the operations performed by the computing device 20 may be performed by the user terminal, and the rest may be performed by the server.

The user terminal may be an electronic device that includes a display device and a device for receiving a user input (for example, a keyboard or a mouse), and includes a memory and a processor. In addition, the display device may be implemented as a touch screen to perform a function of receiving a user input. For example, the user terminal may include a notebook personal computer (PC), a desktop PC, a laptop, a tablet computer, a smartphone, or the like, but one or more embodiments are not limited thereto.

The server may be a device that communicates with an external device (for example, a user terminal). For example, the server may be a device that stores various types of data including medical information and information about machine learning models. For example, the server may be an electronic device that includes a memory and a processor and has self-computing power. For example, the server may be a cloud server or an on-premise server.

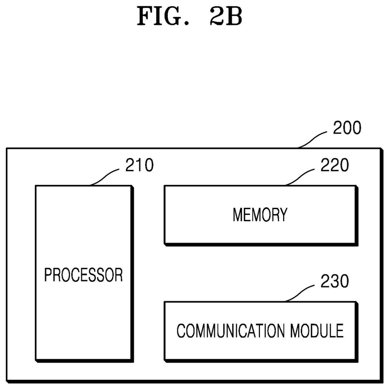

Hereinafter, examples of a user terminal and a server will be described with reference to FIGS. 2A and 2B.

FIG. 2A is a block diagram illustrating an example of a user terminal 100 according to an embodiment.

Referring to FIG. 2A, the user terminal 100 includes a processor 110, a memory 120, an input/output interface 130, and a communication module 140. For convenience of description, only components related to the present disclosure are shown in FIG. 2A. Accordingly, in addition to the components shown in FIG. 2A, other general-purpose components may be further included in the user terminal 100. In addition, it will be apparent to those skilled in the art related to the present disclosure that the processor 110, the memory 120, the input/output interface 130, and the communication module 140 shown in FIG. 2A may be implemented as independent devices.

The processor 110 may process instructions of a computer program by performing a basic arithmetic operation, a logic operation, and an input/output operation. Here, the instruction may be provided from the memory 120 or an external device (for example, a server 200). In addition, the processor 110 may control overall operations of other components included in the user terminal 100.

The processor 110 may generate information about cells and components of the cells expressed in a pathological slide image by analyzing the pathological slide image by using a machine learning model.

For example, the information may include at least one of first information about staining intensity of the cells and the components, second information about a staining level of the cells and the components, and/or third information about polarity of the cells.

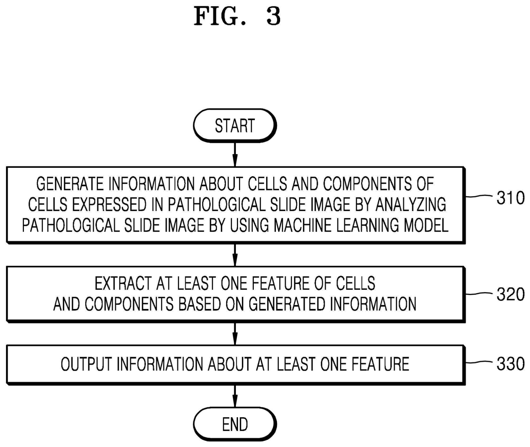

An example in which the processor 110 generates the information about the cells and the components of the cells will be described below with reference to operation 310 of FIG. 3.

The processor 110 may extract at least one feature for the cells and the components based on the generated information.

For example, the processor 110 may extract at least one feature for at least one selected of the cells and the components. Here, the selection may include a first selection based on a user input, a second selection based on a threshold set based on at least one piece of information (for example, the information about the cells and the components of the cells), or a third selection based on any one of types of cells identified through analysis by the machine learning model.

For example, the at least one feature may include at least one of a first feature related to a geometric shape of the cells and the components, a second feature related to staining intensity of the cells and the components, a third feature related to a texture of the cells and the components, a fourth feature based on a combination of some of the first to third features of the components, or a fifth feature based on a combination of at least one of the first to third features of the components and the polarity of the cells.

An example in which the processor 110 extracts at least one feature for the cells and the components of the cells will be described below with reference to operation 320 of FIG. 3.

The processor 110 may output information about at least one feature.

For example, the processor 110 may cluster the cells into any one of a plurality of classes based on at least one feature. Afterwards, the processor 110 may output medical information about a subject corresponding to the pathological slide image based on a result of the clustering.

Examples in which the processor 110 outputs the medical information may vary. As an example, the processor 110 may output categorized information based on a value corresponding to at least one feature. As another example, the processor 110 may output information about at least one feature by overlaying the information on the pathological slide image. As another example, the processor 110 may output information about at least one biomarker corresponding to a specific treatment based on the result of the clustering. As another example, the processor 110 may output information about treatment responsiveness to a specific treatment based on the result of the clustering.

An example in which the processor 110 outputs the information about at least one feature will be described below with reference to operation 330 of FIG. 3.

The processor 110 may be implemented as an array of a plurality of logic gates or may be implemented as a combination of a general-purpose microprocessor and a memory in which a program executable in the microprocessor is stored. For example, the processor 110 may include a general-purpose processor, a central processing unit (CPU), a microprocessor, a digital signal processor (DSP), a controller, a microcontroller, a state machine, or the like. In some environments, the processor 110 may include an application-specific semiconductor (ASIC), a programmable logic device (PLD), a field programmable gate array (FPGA), or the like. For example, the processor 110 may refer to a combination of processing devices such as a combination of a DSP and a microprocessor, a combination of a plurality of microprocessors, a combination of one or more microprocessors in conjunction with a DSP core, or a combination of any other such configurations.

The memory 120 may include any non-transitory computer-readable recording medium. As an example, the memory 120 may include a permanent mass storage device such as a random access memory (RAM), a read-only memory (ROM), a disk drive, a solid state drive (SSD), or a flash memory. As another example, the permanent mass storage device such as a ROM, an SSD, a flash memory, or a disk drive may be a separate permanent storage device which is distinguishable from a memory. In addition, an operating system (OS) and at least one program code (for example, a code through which the processor 110 performs operations to be described below with reference to FIGS. 3 to 12) may be stored in the memory 120.

These software components may be loaded from a computer-readable recording medium separate from the memory 120. The separate computer-readable recording medium may be a recording medium that may be directly connected to the user terminal 100, and may include, for example, a computer-readable recording medium such as a floppy drive, a disk, a tape, a digital video disk (DVD)/compact disc (CD)-ROM drive, or a memory card. For example, the software components may be loaded into the memory 120 through the communication module 140 instead of the computer-readable recording medium. For example, at least one program may be loaded into the memory 120 based on a computer program (for example, a computer program through which the processor 110 performs operations to be described below with reference to FIGS. 3 to 12) installed by files provided through the communication module 140 by developers or a computer file distribution system that distributes installation files of applications.

The input/output interface 130 may be a means for interfacing with a device (for example, a keyboard or a mouse) which may be for input or output and may be connected to or included in the user terminal 100. Although the input/output interface 130 is shown in FIG. 2A as being an element configured separately from the processor 110, one or more embodiments are not limited thereto, and the input/output interface 130 may be included in the processor 110.

The communication module 140 may provide a configuration or function for the server 200 and the user terminal 100 to communicate with each other through a network. In addition, the communication module 140 may provide a configuration or function for the user terminal 100 to communicate with other external devices. For example, a control signal, an instruction, data, or the like, which is provided under the control of the processor 110, may be transmitted to the server 200 and/or an external device through the communication module 140 and a network.

In some embodiments, although not shown in FIG. 2A, the user terminal 100 may further include a display device. For example, the user terminal 100 may be connected to an independent display device through a wired or wireless communication method to transmit or receive data to or from the independent display device. For example, a medical image, medical information, information related to a machine learning model, and the like may be provided to a user through the display device.

FIG. 2B is a block diagram illustrating an example of the server 200 according to an embodiment.

Referring to FIG. 2B, the server 200 includes a processor 210, a memory 220, and a communication module 230. For convenience of description, only components related to the present disclosure are shown in FIG. 2B. Accordingly, in addition to the components shown in FIG. 2B, other general-purpose components may be further included in the server 200. In addition, it will be apparent to those skilled in the art related to the present disclosure that the processor 210, the memory 220, and the communication module 230 shown in FIG. 2B may be implemented as independent devices.

The processor 210 may control the communication module 230 to transmit a pathological slide image to the user terminal 100. For example, the server 200 may receive a pathological slide image from the user terminal 100.

For example, the processor 210 may generate information about cells and components of the cells expressed in the pathological slide image. In addition, the processor 210 may control the communication module 230 to transmit the generated information to the user terminal 100.

For example, the processor 210 may extract at least one feature for the cells and the components based on the generated information. The processor 210 may control the communication module 230 to transmit information about at least one feature to the user terminal 100.

In other words, at least one of the operations of the processor 110 described above with reference to FIG. 2A may be performed by the processor 210. In this case, the user terminal 100 may output information transmitted from the server 200 through the display device.

In some embodiments, an embodiment of the processor 210 is the same as an embodiment of the processor 110 described above with reference to FIG. 2A, and thus a detailed description thereof will be omitted.

Various types of data, such as data generated according to the operation of the processor 210, may be stored in the memory 220. In addition, the memory 220 may store an OS and at least one program (for example, a program required for the processor 210 to operate).

In some embodiments, since an embodiment of the memory 220 is the same as an embodiment of the memory 120 described above with reference to FIG. 2A, a detailed description thereof will be omitted.

The communication module 230 may provide a configuration or function for the server 200 and the user terminal 100 to communicate with each other through a network. In addition, the communication module 230 may provide a configuration or function for the server 200 to communicate with other external devices. For example, a control signal, an instruction, data, or the like, which is provided under the control of the processor 210, may be transmitted to the user terminal 100 and/or an external device through the communication module 230 and a network.

FIG. 3 is a flowchart for describing an example of a method of analyzing a pathological slide image according to an embodiment.

The method shown in FIG. 3 includes operations that are processed in time series by the computing device 20 (100 or 200) or the processor 110 or 210 shown in FIGS. 1 to 2B. Therefore, even if certain contents are omitted below, the described contents of the computing device 20 (100 or 200) or the processors 110 and 210 may also be applied to the method shown in FIG. 3.

In addition, hereinafter, the processor 110 outputting information, an image, or the like includes the processor 110 controlling a display device to output the information, the image, or the like.

In operation 310, the processor 110 may generate information about cells and components of the cells expressed in a pathological slide image by analyzing the pathological slide image by using a machine learning model.

Here, the components of the cells may include a cell membrane, cytoplasm, and a nucleus. In other words, the processor 110 may generate information about the cell expressed in the pathological slide image as well as information about each of the cell membrane, the cytoplasm, and the nucleus included in the cell.

For example, the pathological slide image may be an immunohistochemistry (IHC)-stained image, but is not limited thereto. For example, IHC staining may be a method of visualizing an antibody conjugated with peroxidase through a 3,3'-diaminobenzidine (DAB) reaction, but is not limited thereto. In other words, the generation of the pathological slide image is not limited to a staining type of a specific antibody.

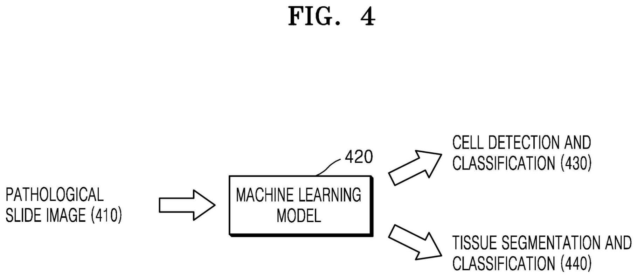

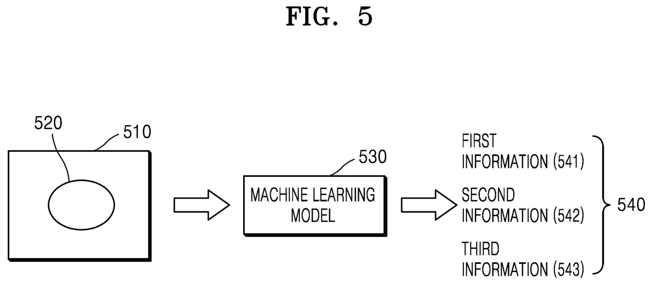

Hereinafter, an example in which the processor 110 analyzes a pathological slide image and generates information about cells and components of the cells expressed in the pathological slide image will be described with reference to FIGS. 4 and 5.

FIG. 4 is a diagram for describing an example in which the processor 110 according to an embodiment analyzes a pathological slide image 410.

Referring to FIG. 4, the processor 110 may analyze the pathological slide image 410 by using a machine learning model 420. For example, the processor 110 may analyze patches obtained by dividing the pathological slide image 410. For example, the processor 110 may divide the pathological slide image 410 into a predetermined size (for example, 1,216×1,216 pixels) to generate patches. The processor 110 may analyze the patches.

Accordingly, the processor 110 may perform detection and classification 430 on cells included in the pathological slide image through the machine learning model 420. In addition, the processor 110 may perform segmentation and classification 440 on tissues included in the pathological slide image through the machine learning model 420.

Hereinafter, an example in which the processor 110 analyzes the pathological slide image 410 by using the machine learning model 420 will be described. In the same manner as in the following analysis method, the processor 110 may analyze the patches.

In an embodiment, the processor 110 may analyze the pathological slide image 410 to perform the segmentation and classification 440 on a plurality of tissues.

For example, by using the machine learning model 420, the processor 110 may output a detection result in the form of layers representing tissues on the pathological slide image 410. In this case, by using learning data including a plurality of reference pathological slide images (or patches) and a plurality of pieces of reference label information, the machine learning model 420 may be trained to detect areas in the pathological slide image 410 corresponding to tissues in the reference pathological slide images.

The processor 110 may perform classification on a plurality of tissues expressed in the pathological slide image 410. For example, the processor 110 may classify the tissues in the pathological slide image 410 into a cancer area or other areas. For example, the processor 110 may classify the tissues in the pathological slide image 410 into any one of a cancer area, a cancer stroma area, a necrosis area, and a background area.

However, an example in which the processor 110 classifies the areas included in the pathological slide image 410 is not limited to the above-described example. In other words, one or more embodiments are not limited to the above-described areas (cancer area, cancer stroma area, necrosis area, and background area), the processor 110 may classify the areas included in the pathological slide image 410 into a plurality of categories based on various criteria. For example, the areas included in the pathological slide image 410 may be classified into a plurality of categories according to preset criteria or criteria set by a user.

In an embodiment, the processor 110 may analyze the pathological slide image 410 to perform the detection and classification 430 on the plurality of cells.

First, the processor 110 may analyze the pathological slide image 410 to detect cells from the pathological slide image 410 and output a detection result in the form of layers representing the cells.

By using the machine learning model 420, the processor 110 may output the detection result in the form of layers representing the cells on the pathological slide image 410. In this case, by using learning data including a plurality of reference pathological slide images (or patches) and a plurality of pieces of reference label information, the machine learning model 420 may be trained to detect locations and types of cells in the reference pathological slide images in the pathological slide image 410.

The processor 110 may perform classification on the plurality of cells included in the pathological slide image 410. For example, the processor 110 may classify the plurality of cells into tumor cells or other cells. For example, the processor 110 may classify the plurality of cells into at least one of tumor cells, lymphocyte cells, fibroblasts, endothelial cells, macrophages, and other cells.

However, an example in which the processor 110 classifies the cells expressed in the pathological slide image 410 is not limited to the above-described example. In other words, one or more embodiments are not limited to the cells described above (that is, the tumor cells, the lymphocyte cells, and other cells), the processor 110 may classify the cells expressed in the pathological slide image 410 into a plurality of categories based on various criteria. The cells of the pathological slide image 410 may be grouped into a plurality of categories according to preset criteria or criteria set by a user.

FIG. 5 is a diagram for describing an example in which the processor 110 according to an embodiment generates information 540 about cells and components of the cells.

Referring to FIG. 5, the processor 110 uses a machine learning model 530 to generate information 540 about cells and components expressed in a pathological slide image 510. Here, the information 540 may include at least one of first information 541 about staining intensity of the cells and the components, second information 542 about a staining level of the cells and the components, and/or third information 543 about polarity of the cells.

For example, the machine learning model 530 may be a model that is identical to or different from the machine learning model 420 of FIG. 4. The processor 110 may generate the information 540 by using an analysis result described above with reference to FIG. 4.

Referring to FIG. 5, the pathological slide image 510 may include a cell 520. Here, the pathological slide image 510 may be one patch obtained by dividing a whole slide image. The processor 110 may identify components included in the cell 520 by analyzing the pathological slide image 510 by using the machine learning model 530. For example, the components may include a cell membrane, cytoplasm, and a nucleus.

As the components are identified in the pathological slide image 510, the processor 110 may output information such as locations and shapes of the components. For example, the machine learning model 530 may be a deep convolution network. In addition, the machine learning model 530 may be trained through fully-supervised learning using reference pathological slide images (or patches), which include manual annotations, as learning data. However, a learning method of the machine learning model 530 is not limited to those described above. For example, the machine learning model 530 may be trained through self-supervised learning using reference pathological slide images, which do not include annotations, as learning data.

As described above with reference to FIG. 4, the processor 110 may accurately identify not only an object (for example, the cell 520) itself from the pathological slide image 510, but also components constituting the object. In addition, as will be described below, the processor 110 may generate the first information 541 about staining intensity of the cell 520 and components of the cell 520 and the second information 542 about a staining level.

The processor 110 may generate the first information 541 based on the pathological slide image 510 and information about components of the object (for example, the cell 520). For example, by using the machine learning model 530, the processor 110 may output the first information 541 about the cell 520 or at least one of the components of the cell 520. For example, the first information 541 may include at least one of a first score corresponding to staining intensity of the cell membrane, a second score corresponding to staining intensity of the cytoplasm, and a third score corresponding to staining intensity of the nucleus.

For example, the machine learning model 530 may be trained based on manual annotations. Manual annotations may be annotations in which an expert assigns a staining class to each of components of a cell (for example, a cell membrane, cytoplasm, and a nucleus). For example, an annotation may be given as any one of four classes (TC0, TC1+, TC2+, and TC3+). In addition, as a non-limiting example, respective classes may correspond to linearly spaced values between 0 and 1. For example, TC0 may be 0, TC1+ may be 0.33, TC2+ may be 0.66, and TC3+ may be 1.0. However, the number of classes and values corresponding to respective classes are not limited to those described above.

In addition, the processor 110 may generate the second information 542 about the cell 520 or any one of the components of the cell 520. For example, by using the machine learning model 530, the processor 110 may output the second information 542 about the cell 520 or any one of the components of the cell 520.

For example, the second information 542 may include a class corresponding to a staining level of the cell 520, the nucleus of the cell 520, the cytoplasm of the cell 520, or the cell membrane of the cell 520. The processor 110 may analyze the pathological slide image 510 to generate the second information 542.

The processor 110 may analyze the pathological slide image 510 to generate information about the components of the cell 520. Accordingly, the processor 110 may generate the second information 542 from the pathological slide image 510 by using the machine learning model 530. That is, the processor 110 may detect the cell 520 from the pathological slide image 510 and may identify a location and staining completeness of each of the nucleus of the cell 520, the cytoplasm of the cell 520, and the cell membrane of the cell 520. Accordingly, the processor 110 may classify cells according to a staining level of a cell, a nucleus, cytoplasm, and a cell membrane.

For example, a class may be any one of three classes (Class 0, Class 1, and Class 2). Specifically, "Class 0" may mean that a cell, a nucleus, cytoplasm, or a cell membrane is negative or not stained, "Class 1" may mean that a cell, a nucleus, cytoplasm, or a cell membrane is partially stained, and "Class 2" may mean that a cell, a nucleus, cytoplasm, or a cell membrane is completely stained. However, the number of classes and criteria for each class are not limited to those described above.

In addition, the processor 110 may generate the third information 543 about polarity of the cell 520. Here, polarity may be any one of an apical pole and a basolateral pole which are defined in relation to a luminal structure. Cells are arranged around a lumen which is an empty space inside the tissue. In this case, the lumen is a cavity inside the tissue through which food, blood, or the like passes. An apical surface of a cell refers to a portion facing a lumen and forming a surface on which the cell is in direct contact with an internal space, a basal surface of the cell refers to a portion oriented toward a basement membrane, and a lateral surface refers to a portion in contact with adjacent cells. In normal epithelial cells, polarity of a cell membrane is maintained, and specific membrane proteins are distributed at set locations (for example, apical surface or basolateral areas). However, in cancer cells, polarity may be disrupted, which may lead to a depolarized state in which proteins are diffused across the entire cell membrane. These changes in polarity-based cell membrane staining patterns may be used as important pathological indicators for evaluating tumor characteristics, malignancy, and treatment responsiveness.

For example, by using the machine learning model 530, the processor 110 may identify the cell 520 adjacent to a lumen from the pathological slide image 510. The processor 110 may define areas of a cell membrane of an identified cell as an apical surface, a lateral surface, and a basolateral surface. The third information 543 about polarity of the cell 520 may include information about which location of the cell membrane is stained. As an example, the third information 543 about the polarity of the cell 520 may include information about whether the cell 520 has at least one of an apical pattern in which an apical area corresponding to an upper surface of the cell 520 is stained, a lateral pattern in which a lateral surface of the cell 520 in contact with an adjacent cell is stained, a basolateral pattern in which a basal surface and a lateral surface are stained continuously, and a depolarized pattern in which polarity is lost and proteins are diffused across the entire cell membrane.

Referring again to FIG. 3, in operation 320, the processor 110 may extract at least one feature for cells and components based on the information generated at operation 310.

For example, the processor 110 may extract at least one feature for at least one selected of the cells and the components. Here, the selection may include a first selection based on a user input, a second selection based on a threshold set based on at least one piece of information (for example, the information about the cells and the components of the cells), or a third selection based on any one of types of cells identified through analysis by a machine learning model.

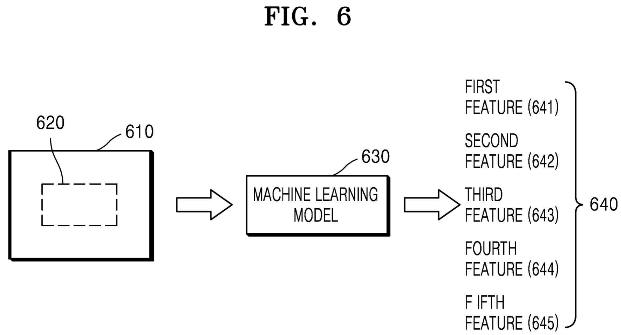

Hereinafter, an example in which the processor 110 selects at least one of cells and components and extracts at least one feature for a selected object will be described with reference to FIG. 6.

FIG. 6 is a diagram for describing an example in which the processor 110 according to an embodiment extracts features 640 for cells and components 620.

Referring to FIG. 6, the processor 110 can extract various features 640 through a machine learning model 630 or a predefined algorithm based on the information generated in operation 320.

In some embodiments, the extraction of the features 640 may be performed on the cells or the components 620 selected from a pathological slide image 610. The processor 110 may select the cells or the components 620 according to any one of the following examples (for example, first to third selections). However, the examples described below are merely illustrative, and a method in which the processor 110 selects the cells or the components 620 is not limited thereto.

As an example, the processor 110 may select the cells or the components 620 based on a user input. Specifically, a user may designate a region of interest (ROI) on the pathological slide image 610. For example, the ROI may be designated by the user marking a box or a circle on the pathological slide image 610. When the user does not designate the ROI, the processor 110 may set all cells detected in the pathological slide image 610 as objects to be analyzed.

As another example, the processor 110 may select the cells or the components 620 based on a threshold set based on at least one piece of information (for example, the information 540 of FIG. 5). For example, the processor 110 may select the cells or the components 620 by applying a threshold based on a staining intensity score (for example, the first information 541 of FIG. 5). Such a selection may be used to secure the reliability of staining texture analysis. For example, the processor 110 may apply a single threshold (for example, 0.XX) to one subcellular area or all subcellular areas to exclude cells with insufficient staining intensity from an object to be analyzed (that is, select only cells with specific staining intensity or more). For example, the processor 110 may apply different thresholds to respective areas included in the pathological slide image 610.

As another example, the processor 110 may select the cells or the components 620 based on any one of types of cells identified by analyzing the pathological slide image 610. For example, the processor 110 may select specific types of cells (for example, tumor cells) from all cells expressed in the pathological slide image 610.

The processor 110 may analyze the cells or the components 620 to extract at least one of the features 640. For example, the features 640 may include at least one of the following examples (for example, first to fifth features 641 to 645). However, the examples described below are merely illustrative examples of features, and the processor 110 may extract other features of the cells or the components 620.

The first feature 641 may be a feature related to a geometric shape of the cells or the components 620. Specifically, the processor 110 may extract statistics representing a shape of a subcellular area as the first feature 641. For example, the first feature 641 may be a standardized measurement value of the cells or the components 620, such as an area, a perimeter, skewness (for example, a major axis/minor axis length or eccentricity), or shape irregularity.

The second feature 642 may be a feature related to staining intensity of the cells and the components 620. Specifically, the processor 110 may extract statistics representing staining intensity as the second feature 642. For example, the processor 110 may calculate mean DAB intensity, a standard deviation, skewness of distribution, upper quantiles (for example, the top 10% of pixel intensities), or the like for a nucleus, cytoplasm, and a cell membrane of each cell (or all cells within a specific area). The processor 110 may regard a calculated value as the second feature 642.

The second feature 642 may reflect intensity (for example, a mean value) and uniformity (for example, a standard deviation or skewness) of protein expression. For example, high staining intensity of a nucleus of an estrogen receptor (ER) may indicate strong expression. In addition, a large standard deviation of staining intensity of the nucleus of the ER may indicate heterogeneous expression in which a strongly stained nucleus and a weakly stained nucleus coexist. Specifically, cell polarity information (for example, the third information 543 of FIG. 5) may be used to additionally define specific intensity-based features. For example, the processor 110 may calculate staining intensity of a specific polar region (for example, an apical or basolateral surface) or may calculate a difference in staining intensity between polar regions.

The third feature 643 may be a feature related to a texture of the cells and the components 620. Specifically, the processor 110 may quantify a spatial arrangement pattern of staining intensity to extract the third feature 643. For example, the processor 110 may measure granularity, smoothness, and repetitiveness of a texture. The processor 110 may regard a measured value as the third feature 643.

For example, the processor 110 may determine a Haralick feature based on a gray-level co-occurrence matrix (GLCM) as the third feature 643. For example, the Haralick feature may include contrast, energy, homogeneity, entropy, or the like of a staining pattern. Here, GLCM-based entropy may represent the randomness of staining intensity. In this case, a higher entropy value may refer to non-uniform staining with many speckled patterns, and a lower entropy value may refer to a uniform pattern.

In addition, the third feature 643 may include wavelet features, local binary patterns, or the like. In some embodiments, the third feature 643 may be defined to include cell polarity information (for example, the third information 543 of FIG. 5).

The fourth feature 644 may be a feature based on a combination of some of the first to third features 641 to 643. Specifically, the processor 110 may calculate a proportion between areas for a specific feature by combining at least some of the first to third features 641 to 643 extracted from each area. For example, the processor 110 may calculate a staining intensity proportion of a nucleus to cytoplasm for a particular biomarker. In this case, the calculated proportion may be a biologically important indicator (for example, a proportion of proteins moved to a nucleus) for a subject corresponding to the pathological slide image 610.

In addition, the processor 110 may calculate an area proportion of a nucleus to cytoplasm or the like. The processor 110 may regard the calculated proportion as the fourth feature 644.

The fifth feature 645 may be a feature based on a combination of at least one of the first to third features 641 to 643 of components and polarity of cells. A specific cell may be identified based on staining intensity (that is, the second feature 642) or a texture feature (that is, the third feature 643) in which cell polarity information (that is, the third information 543 of FIG. 5) is integrated.

For example, human epidermal growth factor receptor 2 (HER2) staining interpretation guidelines for gastric cancer focus on staining of a basolateral membrane (for example, strong complete basolateral or lateral membranous reactivity). Similarly, the processor 110 may identify a basolateral region of a cell and a subcell component of a cell that is a membrane. By applying an intensity score threshold to identified components, the processor 110 may identify cells that meet the above example (for example, the HER2 staining interpretation guidelines). In addition, the processor 110 may define a cell (for example, basolateral cytoplasm stained in a speckled pattern) having a specific texture pattern in a specific polar region.

Referring again to FIG. 3, in operation 330, the processor 110 may output information about at least one feature extracted in operation 320.

The processor 110 may output information about at least one of the features 640 of FIG. 6. For example, the processor 110 may output a pathological slide image as well as information about the features 640. For example, the processor 110 may generate and output a report including the information about the features 640.

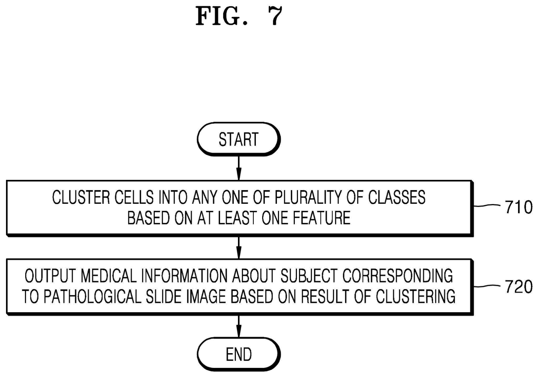

In addition, the processor 110 may output medical information about a subject based on at least one feature. Hereinafter, examples in which the processor 110 outputs medical information about a subject will be described with reference to FIGS. 7 to 11.

FIG. 7 is a flowchart for describing an example in which the processor 110 according to an embodiment outputs medical information about a subject.

In operation 710, the processor 110 may cluster cells into any one of a plurality of classes based on at least one feature.

For example, the processor 110 may cluster cells based on a texture feature of the cells or components (for example, the third feature 643 of FIG. 6).

As an example, the processor 110 may perform unsupervised clustering. Specifically, without predefined labels, the processor 110 may autonomously identify clusters in data based on all subcellular features such as staining patterns and staining intensities.

As another example, the processor 110 may perform supervised clustering. Specifically, a user may manually identify specific types of cells and may label the identified specific types into respective classes. A machine learning model may be trained based on labeled data. The processor 110 may classify the remaining unlabeled cells into respective classes by using the trained machine learning model.

As another example, the processor 110 may perform semi-supervised clustering. For example, a machine learning model may be trained based on both labeled data and unlabeled data.

As another example, the processor 110 may cluster cells in consideration of physical locations of cells expressed in a pathological slide image. The processor 110 may cluster cells in consideration of at least one of a distance between neighboring cells, the number of the neighboring cells, and a feature of an expressed cell.

A result of clustering performed as described above may be used to analyze a relative proportion for each cell type or a spatial distribution of cells (for example, whether the cells are densely concentrated in a certain space or broadly scattered).

In operation 720, the processor 110 may output medical information about a subject corresponding to a pathological slide image based on the result of the clustering.

In this case, forms in which the medical information is output may vary. For example, the processor 110 may output categorized information based on a value corresponding to at least one feature. For example, the processor 110 may output information about at least one feature by overlaying the information on the pathological slide image. For example, the processor 110 may output information about at least one biomarker corresponding to a specific treatment based on the result of the clustering. For example, the processor 110 may output information about treatment responsiveness to a specific treatment based on the result of the clustering.

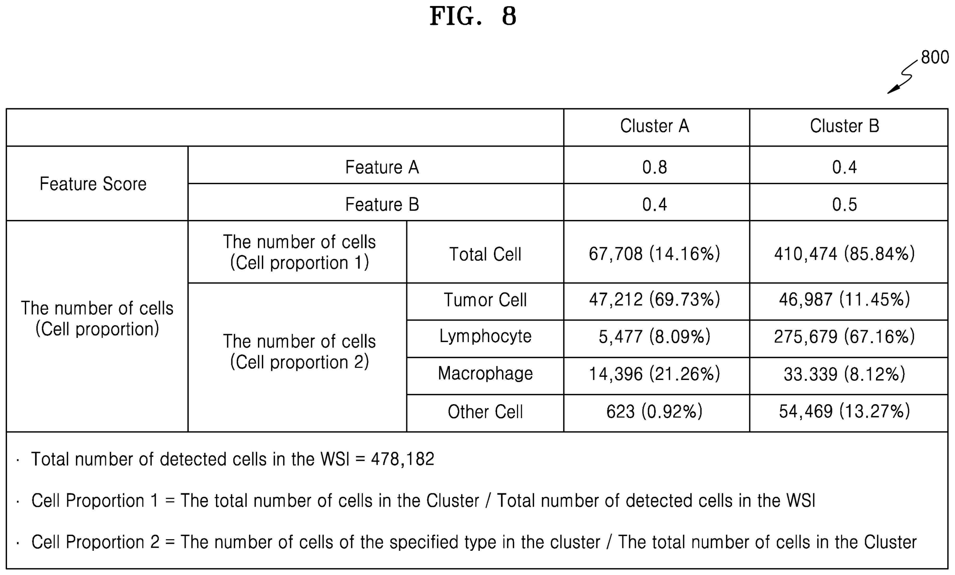

Hereinafter, examples in which the processor 110 outputs medical information about a subject will be described with reference to FIGS. 8 to 11.

FIG. 8 is a diagram for describing an example in which the processor 110 according to an embodiment outputs medical information 800.

Referring to FIG. 8, the processor 110 may output the medical information 800 on a screen of a display device. For example, the medical information 800 may include a slide-level analysis result.

For example, it is assumed that a user has selected cell A and/or cell B, which are to be analyzed and/or clustered, from a pathological slide image. In addition, it is assumed that cell A and cell B have different staining intensities and/or staining patterns.

In this case, if necessary, the user may also select criteria (that is, features) for analyzing and/or clustering cells expressed in the pathological slide image. When the criteria (that is, features) are not selected by the user, the processor 110 may perform clustering using all available features.

For example, the processor 110 may analyze staining intensities and staining patterns of cell A and/or cell B selected by the user to generate Cluster A having staining intensity and staining patterns that are the same as or similar to those of cell A, and Cluster B having staining intensity and staining patterns that are the same as or similar to those of cell B. The processor 110 may output an analysis result (that is, a result of clustering).

For example, the processor 110 may output which feature (that is, at least one of first to fifth features) is used as a basis for classifying Cluster A and cluster B. In FIG. 8, Cluster A and Cluster B are shown as being classified based on "Feature A" and/or "Feature B."

In addition, the processor 110 may output a value corresponding to a distribution of a corresponding feature. Referring to FIG. 8, a value corresponding to a distribution of "Feature A" in Cluster A is output as 0.8.

In addition, the processor 110 may output the number of cells of each type included in each cluster and/or the total number of cells included in each cluster. Referring to FIG. 8, the total number of cells included in Cluster A is output as 67,708. In addition, the number of tumor cells included in Cluster A is output as 47,212, the number of lymphocyte cells is output as 5,477, the number of macrophage cells is output as 14,396, and the number of other cells is output as 623.

In addition, the processor 110 may output a proportion of cells included in each cluster. For example, the proportion may be a value obtained by dividing the number of cells included in a corresponding cluster by the total number of cells detected in the pathological slide image. Referring to FIG. 8, a proportion of cells included in Cluster A is output as 14.16%.

In addition, the processor 110 may output a proportion of cells of each type included in each cluster. For example, the proportion may be a value obtained by dividing the number of cells of a corresponding type included in a corresponding cluster by the total number of cells included in the corresponding cluster. Referring to FIG. 8, a proportion of the tumor cells included in Cluster A is output as 69.73%, a proportion of the lymphocyte cells is output as 8.09%, a proportion of the macrophage cells is output as 21.26%, and a proportion of other cells is output as 0.92%.

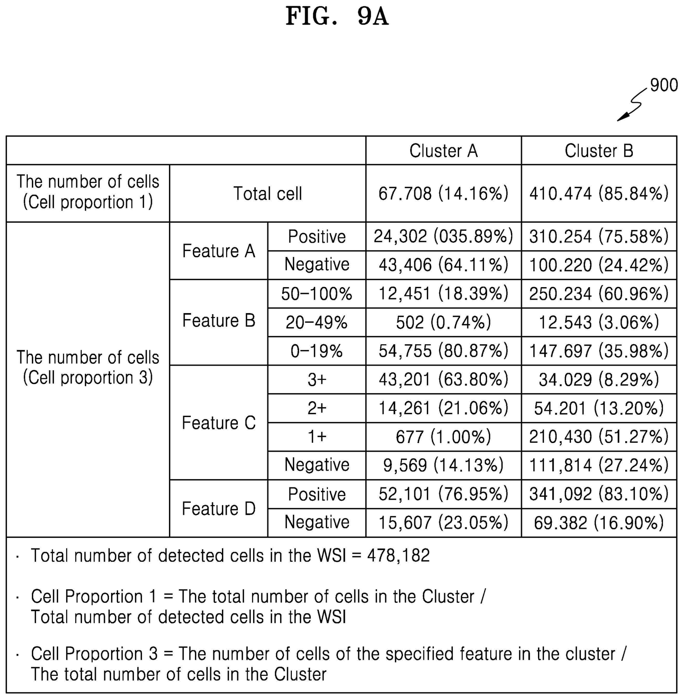

FIG. 9A is a diagram for describing another example in which the processor 110 according to an embodiment outputs medical information 900.

Referring to FIG. 9A, the processor 110 may output the medical information 900 on a screen of a display device. For example, the medical information 900 may include a slide-level analysis result. In this case, the medical information 900 may include categorized information based on a value corresponding to at least one feature.

For example, the processor 110 may determine which category (for example, a level or a positive/negative value) a quantified prediction value for each cell corresponds to. Accordingly, the processor 110 may determine which category each cell corresponds to. Accordingly, the number of cells corresponding to each category and/or a proportion of cells corresponding to each category may be determined.

In this case, a method of setting a category criterion may vary. For example, a category criterion may be set by a user inputting the number of categories to be classified.

For example, it is assumed that the processor 110 has predicted a skewness (or asymmetry) value of a cell. In this case, the processor 110 may quantify corresponding features (for example, values between 0 and 1). When a user inputs that corresponding features are to be classified into four categories and analyzed, the processor 110 may divide a range from 0 to 1 into four sections (for example, level 1 (0 to 0.25), level 2 (0.25 to 0.5), level 3 (0.5 to 0.75), and level 4 (0.75 to 1)). The processor 110 may classify a corresponding cell into one of four categories according to a predicted value of the cell.

A category criterion may be initially set and may be modified or updated as necessary. For example, the processor 110 may recommend a category criterion. For example, the processor 110 may analyze a pathological slide image and may recommend an appropriate number of categories and/or a numerical range of each category. When a user accepts, the category criterion recommended by the processor 110 may be set as a category criterion.

For example, the processor 110 may analyze a pathological slide image and may identify a distribution of prediction values for a specific feature. The processor 110 may subdivide a section, in which predicted values are more densely concentrated, into smaller numerical ranges. In addition, the processor 110 may recommend to a user an appropriate category criterion for a corresponding feature (for example, the number of categories or a numerical range of each category) according to the distribution of the prediction values. When the user accepts, the processor 110 may classify each cell according to the category criterion.

In FIG. 9A, "Feature A" is shown as being classified as being "Positive" and "Negative." In this case, among cells included in Cluster A, the number of cells categorized as being "Positive" is 24,302, and a proportion thereof is output as 35.89%. In some embodiments, among the cells included in Cluster A, the number of cells categorized as being "Negative" is 43,406, and a proportion thereof is output as 64.11%. According to the above-described rules, information about categories constituting each of "Features B to D" may be output.

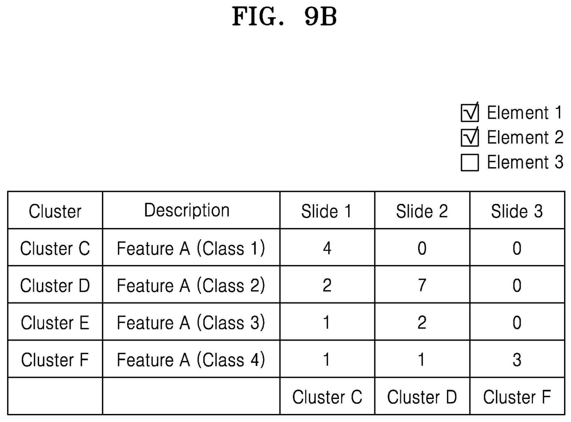

FIG. 9B is a diagram for describing another example in which the processor 110 according to an embodiment outputs medical information 950.

Referring to FIG. 9B, the processor 110 may output the medical information 950 on a screen of a display device. For example, the medical information 950 may include a slide-level analysis result. In this case, the medical information 950 may include information about a result of clustering each cell in consideration of at least one feature of each of cells and physical location information of each of cells.

The processor 110 may analyze and output cells expressed in a pathological slide image in consideration of at least one feature and at least one additional analysis element. In an embodiment, at least one additional analysis element may refer to information identified based on spatial and/or interactive relationships between a cell and a surrounding environment of the cell, and may include at least one of information identified based on physical location information of each of cells (for example, a distance between neighboring cells and the number of the neighboring cells), characteristic information of the neighboring cells (for example, types of the neighboring cells), and information of an area to which a cell belongs.

The processor 110 may determine which category (for example, a class, level, or a positive/negative) a quantified prediction value for each cell corresponds to. In addition, the processor 110 may determine which cluster each of cells belongs to in consideration of a category of each of the cells and physical location information of each of the cells. In addition, the processor 110 may determine the additional analysis element as a preset element or based on a user input.

In FIG. 9B, "Feature A" is shown as being classified into "Class 1," "Class 2," "Class 3," and "Class 4." For example, the processor 110 may determine which class a quantified prediction value for each cell belongs to. In addition, the processor 110 may receive a user input selecting, as additional analysis elements, a distance between neighboring cells (Element 1) and the number of the neighboring cells (Element 2). The processor 110 may determine, as one cell cluster, cells in which a distance between neighboring cells is within a criterion distance, in which the number of the neighboring cells is greater than or equal to a criterion number, and which have identical category information.

In this case, as an example, a criterion distance between neighboring cells may be determined in consideration of at least one of an average size of cells, a size of cells according to a cancer type, a size of cells according to a cell type, and a distance between cells. The criterion distance may be preset or modified by a user. For example, the criterion distance may be set to a value determined by the processor 110 based on features of cells expressed in a pathological slide image, a cell type, a cancer type, and recommended to a user.

As an example, a criterion number of neighboring cells may be set to a value identified based on at least one of a specimen type, cancer type information (for example, characteristics), cell information (for example, a cell class to be analyzed), and a user's analysis purpose (for example, a case in which only large clusters are targeted). The criterion number of neighboring cells needs to be set to a value to secure reliability and reproducibility of interpretation. When interpretation is performed based on too small a number of cells, a possibility of false-positive or false-negative results increases, and thus a minimum number of cells is required for statistically meaningful determination. For example, as a threshold for ensuring the accuracy of HER2 interpretation, the criterion number may be set to five. However, the criterion number may be a fixed value set in advance, or may be set as a recommended value that is dynamically calculated and presented by the processor 110 based on at least one of cell information extracted from a pathological slide image, cancer type information, a specimen type, or a user's analysis purpose. In addition, the criterion number may be modified by a user.

The processor 110 may output the number of clusters included in each pathological slide image. Referring to FIG. 9B, cells included in Slide 1 may be classified into four clusters C, two Clusters D, one Cluster E, and one Cluster F.

In addition, the processor 110 may determine and output a final slide-level analysis result based on the number of clusters. For example, the processor 110 may determine and output a representative cluster of a slide. As another example, the processor 110 may output a value (for example, a score) quantifying diversity of a slide.

As an example, the processor 110 may determine a representative cluster of a slide according to the following rules. For example, when one or more Clusters C consisting of Class 1 are present in a pathological slide image, the processor 110 may determine a representative cluster of the corresponding pathological slide image as Cluster C. When Cluster C consisting of Class 1 is not present, but one or more Clusters D consisting of Class 2 are present in a pathological slide image, the processor 110 may determine a representative cluster of the corresponding pathological slide image as Cluster D. When Cluster C consisting of Class 1 and Cluster D consisting of Class 2 are not present, but one or more Clusters E consisting of Class 3 are present in a pathological slide image, the processor 110 may determine a representative cluster of the pathological slide image as Cluster E. When Cluster C consisting of Class 1, Cluster D consisting of Class 2, and Cluster E consisting of Class 3 are all not present, the processor 110 may determine a representative cluster of a corresponding pathological slide image as Cluster F.

A value quantifying diversity of a slide refers to a value that quantifies how diversely the configuration and distribution of clusters included in a pathological slide image are present. For example, a value quantifying diversity of a slide may be calculated by reflecting types, numbers, proportions, relative sizes, standard deviations, and physical distributions of respective clusters.

The processor 110 may output medical information about a subject corresponding to a pathological slide image by using the final slide-level analysis result. For example, when the value quantifying the diversity of the slide is greater than a criterion value, the processor 110 may determine that treatment responsiveness to a specific treatment is poor and may output the determination.

In some embodiments, although not shown in FIGS. 8 to 9B, the processor 110 may visually distinguish Clusters A to F from each other to output Clusters A to F on a pathological slide image. For example, Clusters A to F may be output to be distinguished from each other by color, brightness, transparency, or the like. Specifically, as similarity increases, color, brightness, transparency, or the like may be output more similarly, and as the similarity decreases, color, brightness, transparency, or the like may be output more differently.

In addition, as shown in FIGS. 8 to 9B, the processor 110 may output medical information in a text form, but may also visualize and output the medical information by using graphs or the like.

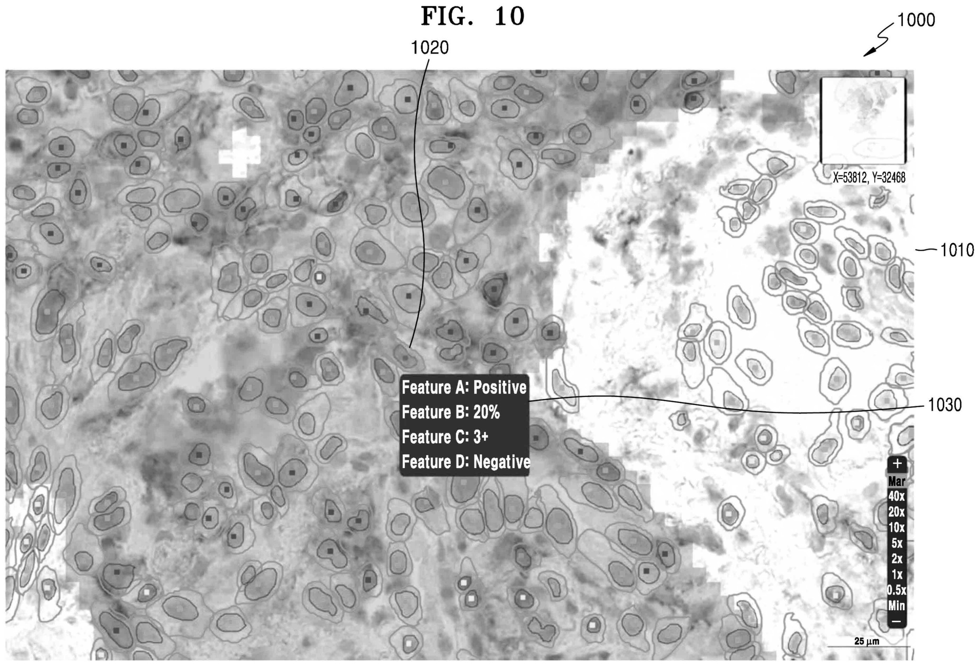

In addition, information about a feature selected by a user may be output at a cell level. An example in which information about a feature is output at a cell level will be described below with reference to FIG. 10.

As described above with reference to FIGS. 8 to 9B, the processor 110 may acquire results of clustering a plurality of subjects. The processor 110 may also acquire information about whether each subject is responsive to a drug (that is, has treatment responsiveness). For example, the processor 110 may compare and analyze results of clustering responders who are responsive to a drug and/or non-responders who are not responsive well to a drug. The processor 110 may output an analysis result.

Specifically, the processor 110 may compare and analyze cell proportion values of the responders. In addition, the processor 110 may determine a biomarker and a cell proportion that serves as a criterion for distinguishing responders from non-responders. Here, the biomarker may be a proportion of cells with cluster features that play an important role in distinguishing responders from non-responders.

In addition, the processor 110 may identify which features cells included in a corresponding cluster have and may determine, as a biomarker, a proportion of cells exhibiting the corresponding features. In the following description, a cell exhibiting a corresponding feature will be referred to as "Cell type X." Here, "X" may be a name of a cluster.

First, a case in which a cell proportion of one Cell type X is determined as a biomarker will be described.

When, as the results of clustering the responders, a certain cluster commonly exhibits a statistically significantly higher or lower cell proportion as compared to the non-responders, the processor 110 may determine which features cells included in a corresponding cluster have. The processor 110 may determine a proportion of cells exhibiting the corresponding features as a biomarker. The processor 110 may determine a criterion value of the cell proportion of Cell type X for distinguishing responders from non-responders.

As an example, the processor 110 may analyze the results of clustering the responders and the non-responders. When a cell proportion of Cluster B is significantly higher in responders than in non-responders, the processor 110 may determine cells exhibiting Feature A and Feature B corresponding to Cluster B as Cell type B. The processor 110 may determine a proportion of Cell type B as a biomarker. The processor 110 may determine a criterion value of the cell proportion of Cell type B for distinguishing responders from non-responders (for example, a cell proportion that serves as a criterion that should be satisfied by the cell proportion of Cluster B).

As another example, the processor 110 may analyze the results of clustering the responders and the non-responders. When the cell proportion of Cluster B is significantly higher in responders than in non-responders and/or when a proportion of cells of a specific type (for example, tumor cells) is high, cells exhibiting Feature A and Feature B corresponding to Cluster B may be determined as Cell type B, and a proportion of Cell type B may be determined as a biomarker. The processor 110 may determine the criterion value of the cell proportion of Cell type B for distinguishing responders from non-responders (for example, a cell proportion that serves as a criterion that should be satisfied by cells of a specific type included in Cluster B).

In some embodiments, it is assumed that a user wants to further subdivide and identify a proportion of cells corresponding to a specific category (for example, 3+ intensity) for one feature (for example, staining intensity of a cell membrane). In this case, the processor 110 may perform clustering and may identify a subset in a specific category. The processor 110 may determine the identified subset as a biomarker.

As an example, when a user selects cell A (a cell with a completely stained cell membrane) and cell B (a false-positive-stained cell or an artifact) in a pathological slide image, the processor 110 may perform clustering such that Cluster A includes cells that are well stained and analyzable, and Cluster B includes cells that are excluded from analysis. Afterwards, when the user analyzes the cells included in Cluster A by subcategorizing specific features, the processor 110 may categorize the corresponding features and may calculate a cell proportion corresponding to each category. The processor 110 may output the calculated cell proportion.

As another example, the processor 110 may perform clustering such that Cluster A includes cells with stained cytoplasm, and Cluster B includes other cells. In this case, the processor 110 may extract a feature indicating whether a specific pattern (for example, a granular cytoplasmic staining pattern) is present in a cytoplasmic staining result. Afterwards, the processor 110 may subcategorize features (for example, positive or negative) and may define cells classified into a specific category as Cell type X. The processor 110 may determine the cell proportion of Cell type X as a biomarker.

In summary, as an example, when the results of clustering the respondents are analyzed, in a case in which a cell proportion of Cluster B has a larger value than that of other clusters and/or a cell proportion of a specific category of a specific feature is greater than that of other cell proportions, the processor 110 may define cells, in which Feature A of Cluster B is positive, as Cell type B. The processor 110 may determine the cell proportion of Cell type B as a biomarker. The processor 110 may determine a criterion value of the cell proportion of Cell type B for distinguishing responders from non-responders (that is, a cell proportion that serves as a criterion for a category that should be satisfied for a subject to be classified as a responder).

As another example, it is assumed that Cluster B includes a plurality of categories of features. In this case, the processor 110 may define, among cells included in Cluster B, cells in which Feature A is positive, cells in which Feature B is in a range of 50% to 100%, cells in which Feature C is 1+, and cells in which Feature D is positive as Cell types Ba to Bd, respectively. The processor 110 may determine a cell proportion of each of Cell types Ba to Bd as a biomarker. The processor 110 may determine a criterion value of the cell proportion of each of Cell types Ba to Bd for distinguishing responders from non-responders (that is, a cell proportion that serves as a criterion for a category that should be satisfied for a subject to be classified as a responder).

In this case, the criterion value may be determined as a mean value of analysis result information of subjects classified as responders, a median value of the analysis result information of the subjects classified as the responders, a minimum value of the analysis result information of the subjects classified as the responders, a maximum value of the analysis result information of the subjects classified as the responders, a minimum value of analysis result information of subjects classified as non-responders, a maximum value of the analysis result information of the subjects classified as the non-responders, a value of a subject whose analysis result information corresponds to a specific upper or lower percentage (%) among the subjects classified as the responders or the non-responders, or the like. However, examples of the criterion value are not limited to those described above.

For example, it is assumed that cell proportions of Cluster B of Subjects P1, P2, and P3 classified as responders are CP1, CP2, and CP3, respectively. In this case, the processor 110 may determine a criterion value (that is, a criterion cell proportion of Cluster B) for classifying a subject as a responder as a minimum value among CP1, CP2, and CP3. In some embodiments, it is assumed that values corresponding to proportions of tumor cells of Subjects P1, P2, and P3 classified as responders are TP1, TP2, and TP3, respectively. In this case, the processor 110 may determine a criterion value (that is, a criterion cell proportion of tumor cells of Cluster B) for classifying a subject as a responder as a maximum value among TP1, TP2, and TP3.

In addition, in determining a criterion value, weights determined in consideration of the importance in which each of features, a specific cell type, or the like affects a treatment effect may be further considered.

Next, a case in which cell proportions of a plurality of Cell types X are determined as biomarkers will be described.

When, as results of clustering responders, a plurality of clusters (for example, Cluster A (including tumor cells with strongly stained cytoplasm) and Cluster B (including non-tumor cells with strongly stained cytoplasm)) are commonly observed in responders, the processor 110 may identify which features cells included in each cluster have. The processor 110 may determine proportions of cells exhibiting corresponding features as biomarkers. Hereinafter, the above-described biomarkers will be referred to as "Cell type A" and "Cell type B." The processor 110 may determine criterion values of cell proportions of Cell type A and Cell type B for distinguishing responders from non-responders.

As an example, the processor 110 may analyze results of clustering responders and non-responders. The processor 110 may determine cells exhibiting Feature A and Feature B corresponding to Cluster A as Cell type A, and may determine lymphocyte cells exhibiting Feature A and Feature B corresponding to Cluster B as Cell type B.

For example, in order for a subject to be determined as a respondent, it is assumed that a proportion of Cell type A of Cluster A should exceed M and a proportion of Cell type B of Cluster B should be less than N. In this case, the processor 110 may determine each of M and N as a criterion value for each biomarker.

As another example, the processor 110 may analyze results of clustering responders and non-responders. The processor 110 may determine tumor cells exhibiting Feature A and Feature B corresponding to Cluster A as Cell type A, and may determine lymphocyte cells having Feature A and Feature B corresponding to Cluster B as Cell type B. The processor 110 may determine a criterion value of a cell proportion of Cell type A and a criterion value of a cell proportion of Cell type B for distinguishing responders from non-responders.

As another example, the processor 110 may analyze results of clustering responders and non-responders. The processor 110 may determine cells in which Feature A is negative among features of Cluster A as Cell type A, and may determine cells in which Feature A is positive among features of Cluster B as Cell type B. The processor 110 may determine a criterion value of a cell proportion of Cell type A and a criterion value of a cell proportion of Cell type B for distinguishing responders from non-responders.

As another example, it is assumed that Cluster A and Cluster B each include a plurality of categories of features. In this case, the processor 110 may define, among cells included in Cluster A, cells in which Feature A is negative, cells in which Feature B is in a range of 0% to 19%, cells in which Feature C is 3+, and cells in which Feature D is positive as Cell types Aa to Ad, respectively. In addition, the processor 110 may define, among cells included in Cluster B, cells in which Feature A is positive, cells in which Feature B is in a range of 50% to 100%, cells in which Feature C is 1+, and cells in which Feature D is positive as Cell types Ba to Bd, respectively. In addition, the processor 110 may determine a criterion value of a cell proportion of each of Cell Types Aa to Ad for distinguishing responders from non-responders. In addition, the processor 110 may determine a criterion value of a cell proportion of each of Cell types Ba to Bd for distinguishing responders from non-responders.

In some embodiments, the examples described above with reference to FIGS. 8 to 9b correspond to examples in which clustering is performed based on cells selected by a user. However, a clustering method is not limited to those described above.

For example, the processor 110 may perform clustering based on the number of clusters input by a user. Specifically, the user may select the number of clusters (for example, three clusters). In this case, if necessary, the user may also select criteria (that is, features) for analyzing and/or clustering cells expressed in a pathological slide image. When criteria (that is, features) are not selected by the user, the processor 110 may perform clustering by using all available features.

The processor 110 may cluster cells expressed in a pathological slide image based on the number of clusters input by the user. For example, the processor 110 may perform clustering based on unsupervised learning, but one or more embodiments are not limited thereto. When the user has performed both input of the number of clusters and selection of features for analyzing cells, the processor 110 may cluster cells expressed in a pathological slide image by using only the input number of clusters and the selected features.

The processor 110 may output a result of clustering. For example, an example of the output is as shown in FIG. 8.

In addition, if necessary, information about features selected by a user may be output at a cell level. An example in which information about a feature is output at a cell level will be described below with reference to FIG. 10.

In addition, the processor 110 may compare and analyze results of clustering responders who are responsive to a drug and/or non-responders who are not responsive to a drug. The processor 110 may output an analysis result.

FIG. 10 is an image for describing another example in which the processor 110 according to an embodiment outputs medical information.

FIG. 10 shows an example 1000 in which information about features selected by a user is output at a cell level on a screen of a display device. For example, the processor 110 may output information 1030 about at least one feature of a specific cell 1020 by overlaying the information 1030 on a pathological slide image 1010. In other words, the processor 110 may output information about at least one feature for each cell.

For example, the information 1030 at a cell level may include information about which category each of features of the cell 1020 corresponds to and/or which value each of the features has. When cells are classified into categories for each of features and output, the processor 110 may compare an analyzed numerical value with criterion information (that is, a category criterion) to determine a category corresponding to the corresponding numerical value. The processor 110 may output information about the determined category.

In addition, according to a category of features, cells on the pathological slide image 1010 may be visualized differently. For example, visualization information displayed on the pathological slide image 1010 may be turned on/off for each category.

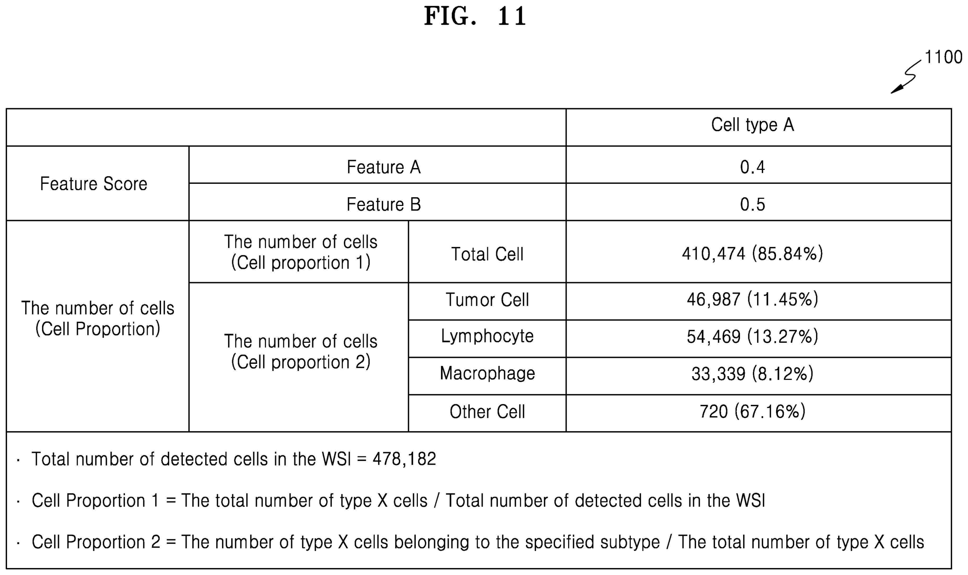

FIG. 11 is a diagram for describing another example in which the processor 110 according to an embodiment outputs medical information 1100.

Referring to FIG. 11, the processor 110 may output the medical information 1100 on a screen of a display device. For example, the medical information 1100 may include information about at least one biomarker corresponding to a specific treatment.

The processor 110 may receive a pathological slide image and may analyze the received image to extract at least one feature for each cell. The processor 110 may determine Cell type X as a biomarker based on at least one feature. The processor 110 may detect cells corresponding to Cell type X from the pathological slide image. The processor 110 may calculate and output the number of the detected cells and/or a proportion of the detected cells. Here, the number of Cell types X may be one or more as described above.

The processor 110 may also output a result of classifying cells for each Cell type X on the pathological slide image. For example, the processor 110 may output the result such that cells are distinguished from each other by different colors, brightness, saturation, or transparency according to cell types. Specifically, as similarity between cell types increases, color, brightness, transparency, or the like may be output more similarly, and as the similarity decreases, color, brightness, transparency, or the like may be output more differently.

In addition, the processor 110 may also output information of Cell type X. For example, the processor 110 may output information about which combination of features is included in Cell type X. In FIG. 11, Cell type X is shown as "Cell type A." In FIG. 11, "Cell type A" is output to include a combination of "Feature A" and "Feature B."

In addition, the processor 110 may also output analysis information about Cell type X. For example, the total number of cells included in Cell type X, a cell proportion of Cell type X, the number of cells for each subtype included in Cell type X (that is, the number of cells for each subtype), a cell proportion for each subtype included in Cell type X, or the like may be output.

Referring to FIG. 11, the total number of cells included in "Cell type A" is output as 410,474, and a cell proportion of "Cell type A" is output as 85.84%. In addition, subtypes of cells included in "Cell type A" are printed as "Tumor Cell," "Lymphocyte," "Macrophage," and "Other Cell." In this case, the number of cells classified as "Tumor Cell" is output as 46,987, the number of cells classified as "Lymphocyte" is output as 54,469, the number of cells classified as "Macrophage" is output as 33,339, and the number of cells classified as "Other Cell" is output as 720. In addition, cell proportions of "Tumor Cell," "Lymphocyte," "Macrophage," and "Other Cell" are output as 11.45%, 13.27%, 8.12%, and 67.16%, respectively.

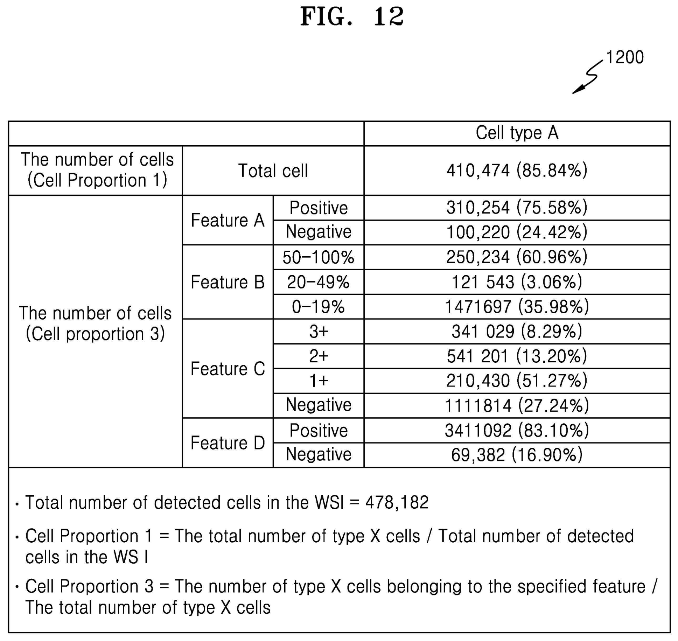

FIG. 12 is a diagram for describing another example in which the processor 110 according to an embodiment outputs medical information 1200.

Referring to FIG. 12, the processor 110 may output the medical information 1200 on a screen of a display device. For example, the medical information 1200 may include information about treatment responsiveness to a specific treatment.

For example, the processor 110 may output the number of Cell types X corresponding to each of categories included in features and/or a proportion of Cell type X corresponding to each of the categories. Here, the number of Cell types X may be one or more as described above.

In FIG. 12, Cell type X is shown as "Cell type A." In FIG. 12, "Cell type A" is output to include a combination of "Features A to D."

Referring to FIG. 12, "Feature A" is classified as being "Positive" and "Negative." In some embodiments, among cells included in "Cell type A," the number of cells categorized as being "Positive" is output as 310,254, and a proportion thereof is output as 75.58%. In some embodiments, among the cells included in "Cell type A," the number of cells categorized as being "Negative" is output as 100,220, and a proportion thereof is output as 24.42%. According to the above-described rules, information about categories constituting each of "Features B to D" may be output.

In addition, the processor 110 may determine whether a subject is responsive to a corresponding drug based on an analysis result. The processor 110 may output a determination result. The processor 110 may compare a cell proportion of Cell type X with a criterion value (that is, a criterion value that should be satisfied for a subject to be classified as a responder). For example, when the cell proportion of Cell type X satisfies a predetermined condition, it may be determined that the subject is responsive to the corresponding drug.

Referring to FIG. 12, the cell proportion of "Cell type A" should satisfy a predetermined condition (for example, the cell proportion should be greater than the criterion value or should be smaller than the criterion value). For example, when the cell proportion of "Cell type A" satisfies a predetermined condition (for example, the cell proportion is K times greater than the criterion value), the processor 110 may determine that the subject is responsive to the corresponding drug.

When there are a plurality of Cell types X, the processor 110 may compare the cell proportion of Cell type A with the criterion value and may compare a cell proportion of Cell type B with the criterion value. When both of the above-described two comparison results satisfy a predetermined condition, the processor 110 may determine that the subject is responsive to the corresponding drug.

As described above with reference to FIGS. 1 to 12, a processing result of the processor 110 may be applied to various applications.

First, not only may the performance of existing machine learning models be improved, but the number of cases determined as false positives may also be reduced.

Machine learning models according to a related art sometimes incorrectly identify staining artifacts as true positives. For example, a machine learning model that detects HER2-positive breast cancer cells may sometimes incorrectly determine cells as false positives due to abrupt dark staining at tissue edges (that is, edge artifacts) or non-specific brown noise in necrotic regions.

A common example of false positive detection is a case in which an apical membrane appears to have strong staining intensity due to non-specific staining of luminal structures. The non-specific staining is not true staining of a cell membrane and should not be regarded as positive staining of a cell.

The processor 110 according to an embodiment may identify an apical membrane in a pathological slide image and may specifically select false positive cells based on a second feature (that is, a feature related to staining intensity) or a third feature (that is, a feature related to a texture). The processor 110 may exclude the selected cells from downstream analysis.

The operation of the processor 110 is not limited to a simple binary classification problem (for example, determining false positives or false negatives), but may also be applied to multi-class classification and regression tasks. That is, the processor 110 may identify cell groups that have been incorrectly predicted by machine learning models according to a related art. The cell groups identified in this way may be used as a new set of labels and used to fine-tune or update the machine learning models according to the related art for improving the performance thereof.

In addition, the operation of the processor 110 enables a human-in-the-loop structure. In addition, within such a structure, new labels usable to improve the performance of the machine learning model may be generated.

Second, QC of IHC analysis may be performed, and antibody optimization may be implemented.

Features according to the present disclosure may also be used as standardized technical indicators for IHC staining quality. Accordingly, the features described in the present disclosure may be used as very useful information not only in clinical laboratories but also in the field of new drug development. Currently, when new antibodies or staining protocols are evaluated, subjective determination by a user is often used to determine how clean or intense staining appears. However, when the features according to the present disclosure are used, staining characteristics may be quantitatively compared between different experimental runs, instruments, or antibody lots.

In addition, in clinical laboratories, benchmark ranges of feature values for control tissues may be established (for example, in ER staining in control tissues, a nuclear positivity rate should exceed 90% and a coefficient of variation (CV) of intensity should be less than 10%). When the features according to the present disclosure are used, staining results that fall outside the above-described criterion ranges may be automatically flagged. As in HER2 or Ki-67, such QC may be particularly important in analyses in which results may be borderline.

In addition, by using the features according to the present disclosure, companies developing IHC reagents may demonstrate that a new automated stainer or polymer detection provides consistency that is equal to or better than that of existing methods. In addition, indicators demonstrating this (for example, a lower spatial variance of staining across the entire slide) may be presented.

Third, it is possible to predict treatment responsiveness.

Predicting a response to a treatment using biomarkers (for example, anti-PD-1 or anti-CTLA-4 in checkpoint inhibitor immunotherapies) is very important. Current approaches mainly focus on the expression levels of specific biomarkers, such as programmed death-ligand 1 (PD-L1) being expressed as a percentage of positive cells (for example, a tumor proportion score (TPS)). For example, current approaches primarily involve grading staining intensity of each cell (for example, HER2 0, 1+, 2+, or 3+) and calculating an overall score that represents the total staining.

In some embodiments, according to the operation of the processor 110 according to an embodiment, beyond a method of quantifying staining intensity, the spatial characteristics of stain updating may be quantified, which indicate fundamental biological differences.

The operation of the processor 110 allows a user (for example, a pathologist) to select cells of interest and automatically identify all cells with similar staining intensities and patterns by using computational clustering methods. A proportion of cells identified in this way (that is, cells of interest) may be used as a key indicator for predicting a treatment response.

For example, the processor 110 may identify a plurality of classes of cells of interest (for example, Class A and Class B) and may combine the plurality of classes to predict treatment responsiveness. For example, when a proportion of Class A-like cells is high and a proportion of Class B-like cells is low, treatment responsiveness may be predicted to be higher.

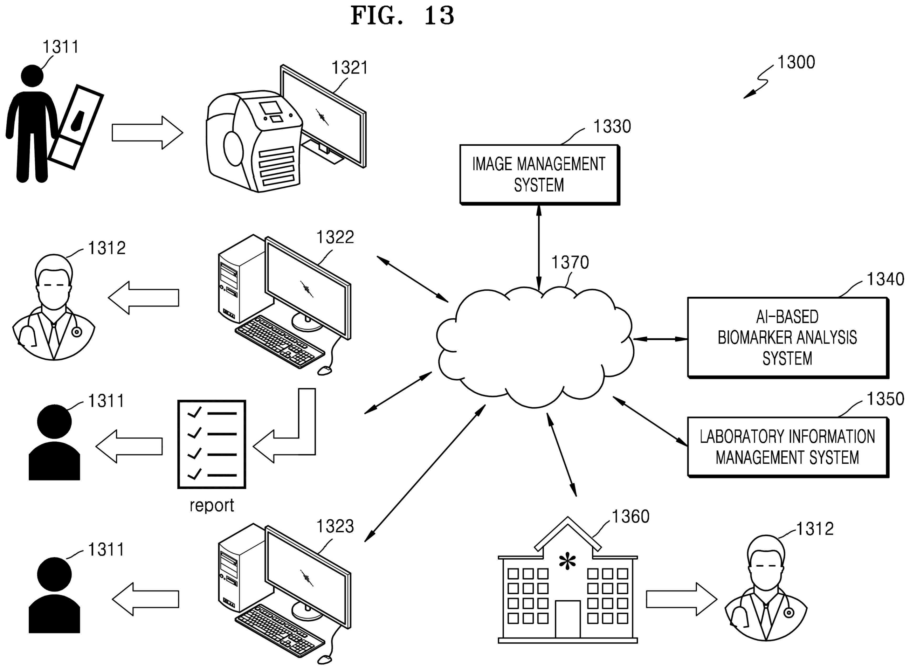

FIG. 13 is a diagram for describing an example of a system 1300 for outputting medical information according to an embodiment.

Referring to FIG. 13, the system 1300 is an example of a system and network for analyzing a pathological slide image by using a machine learning model.

A scanner 1321, user terminals 1322 and 1323, an image management system 1330, an Al-based biomarker analysis system 1340, a laboratory information management system 1350, and/or a hospital or laboratory server 1360 may each be connected to a network 1370, such as the Internet, through one or more computers, servers, and/or mobile devices, or may communicate with a user 1312 through one or more computers and/or mobile devices.

According to various embodiments of the present disclosure, the method described above with reference to FIGS. 2A to 12 may be performed by at least one or a combination of the user terminals 1322 and 1323, the image management system 1330, the Al-based biomarker analysis system 1340, the laboratory information management system 1350, and the hospital or laboratory server 1360.

When a medical image is a pathological slide image, the scanner 1321 may acquire a digitalized image from a tissue sample slide (pathological slide) generated by using a tissue sample of a subject 1311.

The user terminals 1322 and 1323, the image management system 1330, the Al-based biomarker analysis system 1340, the laboratory information management system 1350, and/or the hospital or laboratory server 1360 may generate or acquire, from other devices, tissue samples of one or more subjects 1311, tissue sample slides (pathological slides), digitized images of the tissue sample slides (pathological slides), various types of medical images captured from an object, or any combination thereof. In addition, the user terminals 1322 and 1323, the image management system 1330, the Al-based biomarker analysis system 1340, the laboratory information management system 1350, and/or the hospital or laboratory server 1360 may acquire any combination of subject-specific information of one or more subjects 1311, such as age, medical history, cancer treatment history, family history, past biopsy records, or disease information of the subjects 1311.

The scanner 1321, the user terminals 1322 and 1323, the Al-based biomarker analysis system 1340, the laboratory information management system 1350, and/or the hospital or laboratory server 1360 may transmit medical images, specific information of the subject 1311, and/or a result of analyzing the medical images to the image management system 1330 through the network 1370. The image management system 1330 may include a repository for storing received images, and a storage device for storing an analysis result.

In addition, according to various embodiments of the present disclosure, a machine learning model, which learns and is trained to predict at least one of information about at least one cell, information about at least one area, and medical information (for example, information related to a biomarker, medical diagnosis information, or medical treatment information) from a medical image of the subject 1311, may be stored and operated in the user terminals 1322 and 1323, the image management system 1330, or the like.

As described above, the computing device 20 may apply an approach similar to radiomics, which has been developed for quantitative characterization in radiology, to pathology, thereby providing an interpretable intermediate quantitative feature panel from pathological slide images.

In addition, the computing device 20 may characterize tissues in a consistent manner by generating a standardized library including intensity, texture, and spatial patterns at a subcellular level. In addition, the computing device 20 may contribute to scientific discovery and biomarker verification by serving as a hypothesis generation or verification tool through analysis of a correlation between a specific pattern and a drug response. In addition, according to the computing device 20, cases that have been determined as false positives by machine learning models according to a related art may be reduced, and the performance of the machine learning models may be improved.

In addition, the computing device 20 may provide standardized quantitative indicators for QC and antibody optimization for IHC-stained images. Accordingly, consistency between laboratories or between batches (lots) may be ensured.

In addition, the computing device 20 may quantify subtle subcellular staining patterns beyond simply determining staining intensity of an image. Accordingly, analysis results of the computing device 20 may be used as biomarkers to more precisely predict responsiveness to a specific treatment.

In some embodiments, the above-described method may be recorded as a program that may be executed on a computer and may be implemented in a general-purpose digital computer operating the program using a computer-readable recording medium. In addition, the structure of the data used in the method described above may be recorded on a computer-readable recording medium through various means. Examples of the computer-readable recording medium include storage media such as magnetic storage media (for example, ROMs, RAMs, floppy disks, hard disks, and the like), and optical read media (for example, CD-ROMs and digital videodisks (DVDs)).

It will be understood by those skilled in the art to which the present embodiment pertains that the present disclosure may be implemented in modified forms without departing from the spirit and scope of the present disclosure. Therefore, the disclosed methods should be considered in an illustrative aspect rather than a restrictive aspect. The scope of the present disclosure should be defined by the claims rather than the above-mentioned description, and equivalents to the claims should be interpreted to fall within the present disclosure.

## Claims

1. A computing device comprising:
at least one memory in which at least one command is stored; and
at least one processor operating according to the at least one command,
wherein the at least one processor is configured to generate information about cells and components of the cells expressed in a pathological slide image by analyzing the pathological slide image by using a machine learning model, extract at least one feature for the cells and the components based on the generated information, and control a display device to output information about the at least one feature.

2. The computing device of claim 1, wherein the information includes at least one of first information about staining intensity of the cells and the components, second information about a staining level of the cells and the components, and third information about polarity of the cells.

3. The computing device of claim 1, wherein the at least one processor is further configured to extract the at least one feature for at least one selected from the cells and the components, and
the selection includes a first selection based on a user input, a second selection based on a threshold set based on at least one piece of information, or a third selection based on any one of types of cells identified through the analyzing.

4. The computing device of claim 1, wherein the at least one feature includes at least one of a first feature related to a geometric shape of the cells and the components, a second feature related to staining intensity of the cells and the components, a third feature related to a texture of the cells and the components, a fourth feature based on a combination of some of the first to third features of the components, or a fifth feature based on a combination of at least one of the first to third features of the components and the polarity of the cells.

5. The computing device of claim 1, wherein the at least one processor is further configured to cluster the cells into any one of a plurality of classes based on the at least one feature, and control the display device to output medical information about a subject corresponding to the pathological slide image based on a result of the clustering.

6. The computing device of claim 5, wherein the at least one processor is further configured to control the display device to output categorized information based on a value corresponding to the at least one feature.

7. The computing device of claim 5, wherein the at least one processor is further configured to control the display device to output information about the at least one feature by overlaying the information on the pathological slide image.

8. The computing device of claim 5, wherein the at least one processor is further configured to control the display device to output information about at least one biomarker corresponding to a specific treatment based on the result of the clustering.

9. The computing device of claim 5, wherein the at least one processor is further configured to control the display device to output information about treatment responsiveness to a specific treatment based on the result of the clustering.

10. A method of analyzing a pathological slide image, the method comprising:
generating information about cells and components of the cells expressed in a pathological slide image by analyzing the pathological slide image by using a machine learning model;
extracting at least one feature for the cells and the components based on the generated information; and
outputting information about at least one feature.

11. The method of claim 10, wherein the information includes at least one of first information about staining intensity of the cells and the components, second information about a staining level of the cells and the components, and third information about polarity of the cells.

12. The method of claim 10, wherein the extracting includes extracting the at least one feature for at least one selected from the cells and the components, and
the selection includes a first selection based on a user input, a second selection based on a threshold set based on at least one piece of information, or a third selection based on any one of types of cells identified through the analyzing.

13. The method of claim 10, wherein the at least one feature includes at least one of a first feature related to a geometric shape of the cells and the components, a second feature related to staining intensity of the cells and the components, a third feature related to a texture of the cells and the components, a fourth feature based on a combination of some of the first to third features of the components, or a fifth feature based on a combination of at least one of the first to third features of the components and the polarity of the cells.

14. The method of claim 10, wherein the outputting include:
clustering the cells into any one of a plurality of classes based on the at least one feature; and
outputting medical information about a subject corresponding to the pathological slide image based on a result of the clustering.

15. A computer-readable recording medium having recorded thereon a program for causing a computer to execute the method of claim 10.
